# EUROPEAN PATENT SPECIFICATION

(11) **EP 1 937 245 B1**
(45) Date of publication and mention of the grant of the patent: **27.04.2011**
(21) Application number: 06807318.8
(22) Date of filing: 16.10.2006
(51) Int. Cl.: A61K 31/205, A61P 9/00

(54) **COMPOUND USEFUL FOR THE PREVENTION AND TREATMENT OF LEFT VENTRICULAR HYPERTROPHY IN DIALYSED PATIENTS**
VERBINDUNG ZUR PRÄVENTION UND BEHANDLUNG VON LINKSVENTRIKULÄRER HYPERTROPHIE BEI DIALYSE-PATIENTEN
COMPOSE UTILE POUR LA PREVENTION ET LE TRAITEMENT DE L'HYPERTROPHIE VENTRICULAIRE GAUCHE CHEZ DES PATIENTS DIALYSES

(30) Priority: 17.10.2005 US 250634
(43) Date of publication of application: 02.07.2008
(73) Proprietor: SIGMA-TAU Industrie Farmaceutiche Riunite S.p.A., 00144 Roma (IT)
(72) Inventor: CARMINATI, Paolo, I-20121 Milan (IT); CORSI, Marco, I-00151 Rome (IT)
(74) Representative: HOFFMANN EITLE
(86) International application number: PCT/EP2006/067465
(87) International publication number: WO 2007/045639

(56) References cited:
- WO-A-01/26649
- US-A- 4 237 167
- DATABASE EMBASE ELSEVIER SCIENCE PUBLISHERS, AMSTERDAM, NL; 21 July 2005 (2005-07-21), WANIC-KOSSOWSKA, M. ET AL.: "Influence of L-carnitine on left ventricular mass, its systolic and diastolic function, circadian blood pressure profile and heart rate variability in chronic renal failure patients undergoing hemodialysis therapy" XP002418803
- CAMPISTOL, J. M.: "Uremic myopathy" KIDNEY INTERNATIONAL, vol. 62, 2002, pages 1901-1913, XP002417527
- MATSUMOTO,Y. ET AL.: "Effects of L-Carnitine supplementation on cardiac morbidity in hemodialyzed patients" AM J NEPHROL, vol. 20, 2000, pages 201-207, XP009077860
- DAVIS, T. ET AL.: "Carnitine treatment improved quality of life and erythropoietin doses in selected hemodialysis patients" J AM SOC NEPHROL, vol. 14, November 2003 (2003-11), pages 496A-497A, XP009077832

## Description

The present invention relates to the use of propionyl L-carnitine for the prevention and/or the treatment of left ventricular hypertrophy (LVH) in end-stage renal disease (ESRD) dialysed patients.

During their life, patients suffering from renal insufficiency and on chronic dialysis are exposed to a variety of factors that form the basis for the renal disease-induced heart failure. Among these, artero-venous shunt, uraemic toxins, anaemia, hyperparathyroidism, hyperlipidemia, volume overload and pressure overload, contribute to the development of heart volume and contractility alterations of different severity in each patient.

Age (it is known that the age of patients on dialysis increases continuously and many of them are elder than 70), duration of dialysis and pre-existing renal diseases are additional aggravating factors, as well as possible metabolic impairments that - at cardiac level - are expression of the uremic dysmetabolism.

It is clear that the prevention and/or the treatment of these metabolic impairments represents the most rational approach towards prevention of heart failure induced by renal insufficiency whose relevance as a cause for morbility and mortality in patients on dialysis becomes more and more important.

The clinical course of left ventricular hypertrophy in strictly related to chronic renal failure. In fact, in Miner. Electrolyte Metab. 1999 Jan-Apr; 25(1-2):90-4 is reported that Left ventricular hypertrophy is a very frequent complication in end-stage renal disease patients, and its frequency tends to be even higher in dialyzed patients.

In Nephron 2002 Mar; 90(3):256-61 is reported that LVH commonly occurs in patients with ESRD and is an independent risk factor for cardiovascular events.

In Pediatr Nephrol 2000 Sep; 14(10-11):898-902, is reported that left ventricular hypertrophy (LVH) has been recognized as an independent risk factor for cardiovascular morbidity and mortality in adults with end-stage renal disease.

In Nephron 1990; 55(2):114-20 is reported that a prospective study on the clinical and echocardiographic outcome of left ventricular hypertrophy was undertaken in nondiabetic dialysis patients without dilated cardiomyopathy, who were followed for 3-5 years. After this period 33% of patients had normal echocardiogram, 41% mild and 27% severe hypertrophy (left ventricular wall thickness). In this study is concluded that severe left ventricular hypertrophy occurs frequently in dialysis patients and is associated with a high mortality.

Usually, LVH is detected after high blood pressure is diagnosed, although recent reports indicate that LVH may be present before high blood pressure develops, or may be aggravated by a second condition such as atherosclerosis or diabetes mellitus. LVH is diagnosed by several methods, including electrocardiography, wherein enhanced voltage is detected, by chest X-rays, or preferably by echocardiography, which detects increased myocardial wall thickness and mass.

To date the treatment of LVH in patients not affected by renal insufficiency consists of control of arterial blood pressure, for example by administering one or more of a variety of drugs: diuretics such as diazoxide or hydrochlorthiazide; hypotensives such as methyldopa or hydralazine; beta-adrenergic blockers such as propranolol or labetalol; calcium antagonists such as diltiazem or nifedepine; or angiotensin converting enzyme (ACE) inhibitors such as captopril, spirapril or cilazapril. Many other drugs routinely prescribed to treat essential hypertension, e.g. diuretics and hydralazine, either have no effect on LVH.

Propionyl L-carnitine is a known compound and the process for its preparation is described in US 4,254,053.

The medical use of propionyl L-carnitine is already known.

EP 0793962 discloses the use of propionyl L-carnitine and its pharmacologically acceptable salts for producing a medicament for treating patients suffering from chronic arteriosclerosis obliterans at stage II of Leriche Fontaine's classification.

US 4,434,816 discloses the use of acyl derivatives of DL-, D- or L-carnitine wherein the acyl group contains 2-20 carbon atoms (such as e.g. acetyl, propionyl, butyryl and acetoacetylcarnitine) for treating peripheral vasculopathies.

US 4,968,719 discloses the use of L-carnitine and pharmacologically acceptable salts thereof for treating peripheral vasculopathies.

The Italian Patent IT-1155772 discloses the use of acyl derivatives of L-carnitine (such as e.g. acetyl, propionyl, butyryl) for treating cardiac anoxia, ischaemia, arhythmic syndrome and cardiac insufficiency.

US 4272549 discloses a therapeutic method for the treatment of chronic uraemic patients under periodic haemodialysis which comprises orally administering to such patients, both during the days of haemodialysis session, and during the days between one session an acyl-carnitine (such as e.g. acetyl, propionyl, butyryl-L-carnitine) .

In J. Physiol. 1994 Jun; 266(6 Pt 2) : H 2190-7 is described the use of propionyl L-carnitine for the treatment of left ventricular hypertrophy caused by aortic constriction in rats. The experimental model adopted in this study relates to hypertensive rats *without* renal damages.

In Am. J. Physiol. 1993 Apr; 264(4 Pt 2) : H 1111-7 is described the use of propionyl L-carnitine for the treatment of myocardial infarction in rats. The results reported in this publication shown that propionyl L-carnitine reduces the magnitude of decompensated eccentric hypertrophy produced by myocardial infarction in a manner similar to that found with ACE inhibition. The experimental model adopted in this study relates to myocardial infarction in rats *without* renal damages. In this model propionyl L-carnitine is as active as the ACE inhibitor *which is not active for treating LVH* in patients having renal insufficiency (this aspect will be better discussed in the following).

In. Mol. Cell Biochem. 1992 Oct 21;116(1-2):139-45 is described the metabolic effects of propionyl L-carnitine in the isolated perfused hypertrophied rat heart, in which the hypertrophy was induced by pressure overload. The experimental model adopted in this study relates to the isolated perfused hypertrophied heart of rats *without* renal damages.

In J. Cardiovasc. Pharmacol. 1992 Jul;20(1):88-98 is described the metabolic activities of propionyl-L-carnitine in rats with pressure-overload cardiac hypertrophy. Also the experimental model adopted in this study relates to hypertrophied heart of rats *without* renal damages.

All these studies about the use of propionyl L-carnitine for treating cardiac alterations caused by hypertension or ischemia were carried out in subject having *normal* renal function, and in these models is also recognised the efficacy of drugs such as ACE inhibitors and other anti hypertensive drugs.

The above mentioned publications do not anticipate nor suggest the use of propionyl L-carnitine for the prevention and/or treatment of LVH in uraemic patients. In fact, it is well known that drugs useful for treating LVH in non uraemic patients are ineffective for treating LVH in patients with ESRD or in dialysis.

In Nephrol. Dial. Transplant (1998) 13: 1489-1493 it is reported that LVH in haemodialytic patients is mostly persistent and progressive despite blood pressure reduction by antihypertensive drugs and/or dialysis, and the changes in myocardial histology in LVH are (partly) irreversible. In this publication is also reported that LVH in renal patients is caused by factor other than hypertension, an example of these factors are hyperparathyroidism, sympathetic activity (N. Engl. J. Med. 1992; 327: 1912-1918) reflections of he pulse wave due to stiffened arteries (Kidney Int. 1993; 43 Suppl. 41: 42-49) and anaemia.

M. Wanic-Kossowska et al. in Nadcisnienie Tetnicze, Vol. 9, No. 2, 2005, pp. 103-111 report on their study concerning the influence of L-carnitine treatment on the left ventricular mass. Specifically, as can be seen from the summary of the scientific publication, the study was performed in patients under hemodialysis therapy, and a positive influence of L-carnitine treatment on regression of left ventricular hypertrophy was found.

J.M. Campistol is dealing in Kidney International, Vol. 62, 2002, pp. 1901-1913 with uremic myopathy. In one case described in the article, carnitine was administered to a man with end-stage renal disease after hemodialysis.

The paper from Y. Matsumoto in Am. J. Nephrol, Vol. 20, 2000, pp. 201-207 is concerned with the effects of L-carnitine supplementation on cardiac morbidity in hemodialyzed patients. It is reported in the paper that significant enlarged left ventricular mass before treatment was reduced by 20% after oral L-carnitine treatment.

Davis et al. in J. Am. Soc. Nephrol, Vol. 14, 2003, pp. 496A-497A describe the carnitine treatment of hemodialysis patients showing left ventricular hypertrophy.

In Am. J. Kidney Dis. 2002 Feb; 39(2) : 227-44 is reported that antihypertensive medications alone do not adequately control blood pressure (BP) in hemodialysis patients.

In Ter. Arkh. 1997; 69(6) : 24-7 is reported that the results obtained in a study on the effect captopril versus enalapril on left ventricular (LV) muscle mass and LV systolic and diastolic function in 58 patients with primary glomerulonephritis and moderate chronic renal failure shown that LV ejection fraction, early and late transmitral flow velocities and early to late LV inflow velocities ratio were not significantly affected by both ACE inhibitors.

It is evident to any Expert of the art that LVH caused by hypertension or ischemia, in non uraemic patients is different from LVH in uraemic patients, either from the aetiological point of view or from the therapeutical point of view. It is also known by the Expert of the art that to date does not exist a valid method of treatment of LVH in patients having renal diseases.

In fact, as above mentioned, the development of LVH in ESRD patients is more frequent respect to the frequency in non renal diseased patients (Miner. Electrolyte Metab. 99 Jan-Apr; Nephron 2002; Pediatr. Nephrol. 2000; Nephrol. Dial. Transpl. 97; Blood Perif. 94; Nephron. 90); the aetiology of LVH in ESRD patients is linked to hyperparathyroidism, Sympathetic activity (N.Engl. J. Med. 1992; 327: 1912-1918), reflections of the pulse wave due to stiffened arteries (Kidney Int. 1993; 43 Suppl. 41: 42-49), anaemia, hyperlipaemia, uraemic toxins and metabolic alteration; antihypertensive medications do not adequately control blood pressure in hemodialysis patients (Am. J. Kidney Dis. 2002 Feb; 39(2):227-44, Ter. Arkh. 1997; 69(6):24-7; Nephrol. Dial. Transp1. 97; Nephrol. Dial. Transpl. 98; Am. J. Kidney Dis. 2002).

The knowledge that drugs useful for treating LVH in patients not affected by renal insufficiency such as diuretics, ACE inhibitors or many other drugs routinely prescribed to treat essential hypertension have not effect for treating LVH in patients having renal insufficiency is a strong suggestion that other compounds such as propionyl L-carnitine would not have been effective for treating LVH in patients having renal insufficiency.

Applicant despite this technical prejudice continued to study the effect of propionyl L-carnitine for preventing and/or treating LVH in patients having renal insufficiency.

It has now surprisingly found that propionyl L-carnitine is useful either for the prevention or for the treatment of left ventricular hypertrophy in ESRD or dialysed patients with or without high blood pressure, without having an effect on high blood pressure resulting from essential hypertension.

It is therefore an object of the present invention the use of propionyl L-carnitine, or a pharmaceutical salt thereof, for preparing a medicament for the prevention and/or treatment of left ventricular hypertrophy in ESRD or dialysed patients.

It is a further object of the present invention the use of propionyl L-carnitine for preparing a medicament for the prevention and/or treatment of left ventricular hypertrophy in ESRD or dialysed patients, in which in said patients essential hypertension is not present or is not severe enough to require drug therapy.

It is a further object of the present invention the use of propionyl L-carnitine for preparing a medicament for the prevention and/or treatment of left ventricular hypertrophy in ESRD or dialysed patients, in which in said patients essential hypertension is present.

It is a further object of the present invention the use of propionyl L-carnitine for preparing a medicament for the prevention and/or treatment of left ventricular hypertrophy in ESRD or dialysed patients, in which said propionyl L-carnitine can be used in conjunction with anti hypertensive drugs which do not themselves treat LVH.

It is a further object of the present invention the use of propionyl L-carnitine for preparing a medicament for the prevention and/or treatment of left ventricular hypertrophy in ESRD or dialysed patients, in which said propionyl L-carnitine is in combination with an anti hypertensive agent selected from the group comprising an angiotensin converting enzyme inhibitor or a calcium channel blocker.

What is meant by a pharmacologically acceptable salt of propionyl L-carnitine is any salt of the latter with an acid that does not give rise to unwanted toxic or side effects.

For pharmaceutical salt of propionyl L-carnitine is also intended an FDA approved salts listed in Int. J. of Pharm. 33 (1986), 201-217.

These acids are well known to pharmacologists and to experts in pharmacy, non-limiting examples of such salts are: chloride, bromide, orotate, aspartate, acid aspartate, acid citrate, magnesium citrate, phosphate, acid phosphate, fumarate, and acid fumarate, magnesium fumarate, lactate, maleate and acid maleate, oxalate, acid oxalate, pamoate, acid pamoate, sulphate, acid sulphate, glucose phosphate, tartrate, and acid tartrate, glicerophosphate, mucate, magnesium tartrate, 2-amino etansulphonate, magnesium 2-amino etansulphonate, methansulphonate, coline tartrate, trichloroacetate, and trifluoroacetate.

When propionyl L-carnitine is administered in combination with other anti hypertensive agents, preferred antihypertensives are ACE inhibitors and calcium antagonists. Preferred ACE inhibitors are spirapril, enalapril, ramipril, perindopril, indolapril, lysinopril, quinapril, pentopril, cilazapril, captopril, zofenopril, pivalopril and fosinopril.

Preferred calcium antagonists are diltiazem, nifedipine, verapamil, nicardipine and nimodipine.

The effectiveness of propionyl L-carnitine in preventing and/or treating LVH in dialysed patients has been demonstrated in a preliminary study.

The following example illustrates the invention.

A clinical trial (multicentre, parallel, randomized, double-blind, dose-ranging placebo-controlled study in 300 patients) useful for evaluating both preventive and/or curative effect of the treatment with propionyl L-carnitine (3 and 6 mg/kg given intravenously three times a week at the end of the hemodyalisis session for 26 weeks) has been done.

### Inclusion Criteria

1) male or female out-patients aged > 18 years, suffering from ESRD on bicarbonate dialysis three times a week;
2) patients who have reached a certain degree of dialytic stability (at least since six months on dialysis), so that the variability due to the progressive patient adaptation of the therapeutic treatment is controlled, presenting no clinical signs of hydric overload at the end of dialysis;
3) patients showing a degree of cardiac deterioration defined as inter-ventricular septum and left ventricular wall thickness ≥11 mm;
4) patients with left ventricular ejection fraction >40%;
5) patients under stable pharmacological treatment for at least three months;
6) absence of acute ischaemic events (effort angina, unstable angina, AMI) during the last three months;
7) presence of an adequate acoustic window and a good echogenicity;
8) capacity of co-operating with the administered questionnaire.

### Exclusion Criteria

The patients were not be admitted to the study if they met any of the following exclusion criteria:
1) patients with signs of severe anaemia [haematocrit <28% at least in one evaluation during the three months preceding the study and without evident reasons except those of the normal disease evolution (e.g. bleeding)];
2) patients with evident signs of hyperparathyroidism (plasmatic parathormone values >800 pg/ml; determination of the parameter will be centralised in a single laboratory);
3) patients with body weight>100 kg;
4) patients with bone decalcification at high risk of fractures;
5) patients with proximal artero-venous fistula at the arm;
6) patients with a central venous catheter;
7) patients with previous myocardial infarction (in the three months prior to patient's selection), effort angina or unstable angina;
8) patients with atrial fibrillation or frequent extrasystolic arrhythmia (so to make difficult the recording of ten consecutive cycles in sinus rhythm);
9) haemodinamically significant valvular diseases(moderate-severe);
10) presence of pericarditis and/or evident pericardiac effusion;
11) patients with immune systemic diseases which caused the renal insufficiency;
12) patients with pace-maker;
13) not controllable arterial hypertension (pressure values permanently higher than SAP > 180 mmHg and DAP >100 mmHg);
14) patients treated with LC or its ester derivatives in the three months prior to study start;
15) pregnancy ascertained or presumed, nursing;
16) participation in an other clinical trial in the three months prior to enrolment.

During the whole Randomised Treatment Period, all drugs considered indispensable for the treatment of the disease under study could be administrated providing that they were administered for at least three months prior to patient's enrolment, and that the dosage of those drugs remained stable throughout the whole treatment period.

These drugs comprised eritropoietine, cortisones, antiplatelet drugs, vitamin D and multivitamin complexes, iron-based solutions.

Likewise, treatment with drugs of known effect on cardiac dynamics were allowed with similar restrictions:
ACE-inhibitors or antagonists of angiotensin II receptors
α e β-blockers;
Ca²⁺-antagonists (verapamil, nisoldipina, etc.);
Digitalis.

At the end of the 26^{th} treatment weeks a preliminary analysis (ANOVA variance analysis) was carried out in order to calculate the differences, both between final evaluation and baseline (T₂₆ vs To) and between treated versus control group, of the following signs:
(a) variations induced on the left-ventricular septum thickness; on the posterior left-ventricular wall thickness; on end-systolic and end-diastolic left-ventricular size (1-dimensional echocardiography);
(b) variations induced on end-systolic and end-diastolic left-ventricular volume;
(c) left-ventricular ejection fraction (2-dimensional echocardiography) ;
(d) post-dialytic asthenia by means of Visual Analogue Scale.

The preliminary data obtained have shown that the compound according to the present invention is useful for treating the signs of LVH in the treated patients [signs (a)-(c) above reported].

More surprisingly was to discover that the compound according to the present invention was able to reduce the progression of the signs of LVH [points (a)-(c) above reported] in the treated group respect to its base line. In the control group these signs, respect to its base line, worsened

A variety of pharmaceutical dosage forms are suitable for propionyl L-carnitine, preferably for oral or parenteral administration, although mechanical delivery systems such as transdermal dosage forms are also contemplated.

The typical oral daily dosage of the propionyl L-carnitine for treatment or prevention of LVH is about 1 g to about 5 g, preferred are 2-4 g per day, administered in single or divided doses.

Generally, in treating dialysed patients suffering from LVH or in preventing LVH, the propionyl L-carnitine according to the present invention can be administered intravenously in a preferred daily dosage ranging from about 1 to 24 mg/Kg/body weight, preferred 3-6 mg/Kg/body weight, at the end of each dialysis session.

Typical oral formulations for drugs used in this invention include tablets, capsules, syrups, elixirs and suspensions. Typical injectable formulations for drugs used in this invention include solutions and suspensions.

A variety of pharmaceutical dosage forms are suitable for propionyl L-carnitine, preferably for oral or parenteral administration, although mechanical delivery systems such as transdermal dosage forms are also contemplated.

Since the present invention relates to a use for treating or preventing LVH in dialysed patients, with a combination of active ingredients i.e. propionyl L-carnitine, and an antihypertensive agent, wherein said active ingredients may be administered separately, the invention also relates to combining separate pharmaceutical compositions in kit form. That is, a kit which combines two separate units, a propionyl L-carnitine, composition and an anti hypertensive composition (particularly an ACE inhibitor or a calcium antagonist composition), in one package is contemplated. The kit form is particularly advantageous when the separate components must be administered in different dosage forms (e.g. oral and parenteral) or are administered at different dosage intervals.

## Claims

1. Use of propionyl L-carnitine, or a pharmaceutical salt thereof, for preparing a medicament for the prevention and/or treatment of left ventricular hypertrophy in ESRD or dialysed patients.

2. The use according to claim 1, in which the pharmaceutical salt of propionyl L-carnitine is selected from the group consisting of chloride, bromide, orotate, aspartate, acid aspartate, acid citrate, magnesium citrate, phosphate, acid phosphate, fumarate, and acid fumarate, magnesium fumarate, lactate, maleate and acid maleate, oxalate, acid oxalate, pamoate and acid pamoate, sulphate and acid sulphate, glucose phosphate, tartrate and acid tartrate, glicerophosphate, mucate, magnesium tartrate, 2-amino etansulphonate, magnesium 2-amino etansulphonate, methansulphonate, coline tartrate, trichloroacetate, and trifluoroacetate.

3. The use according to claim 1, in which propionyl L-carnitine is administered when essential hypertension is not present or is not severe enough to require drug therapy.

4. The use according to claim 1, in which propionyl L-carnitine is administered when essential hypertension is present.

5. The use according to claim 1, in which propionyl L-carnitine is used in combination with anti hypertensive agents which do not themselves treat LVH.

6. The use according to claim 5, in which the anti hypertensive agent is selected from the group consisting of angiotensin converting enzyme inhibitor, calcium channel blocker, ACE inhibitors, diuretics, beta-adrenergic blockers or calcium antagonists.

7. The use according to claim 6, in which the ACE inhibitor is selected from the group consisting of spirapril, enalapril, ramipril, perindopril, indolapril, lysinopril, quinapril, pentopril, cilazapril, captopril, zofenopril, pivalopril and fosinopril.

8. The use according to claim 6, in which the calcium antagonists is selected from the group consisting of diltiazem, nifedipine, verapamil, nicardipine and nimodipine.

9. The use according to claim 1, in which propionyl L-carnitine is in a form suitable for enteral or parenteral administration.

10. The use according to claim 1, in which propionyl L-carnitine is administered in a single or divided doses of 1 to about 5 g, preferred are 2-4 g/day when administered by oral route, or from about 1 to 24 mg/Kg/body weight, preferred 3-6 mg/Kg/body weight, at the end of each dialysis session, when administered intravenously.

11. The use according to claim 9, in which propionyl L-carnitine for enteral or parenteral administration is in the form of tablets, capsules, syrups, elixirs, suppositories, suspensions or solutions.

12. The use according to claim 5, in which the combination can be administered from a single pharmaceutical composition which combines the active ingredients in a pharmaceutically acceptable excipient and/or carrier.

13. The use according to claim 5, in which the two active ingredients can be administered separately in a parallel or sequential course.

14. The use according to claim 13, in which the two active ingredients may be administered in any suitable combination of dosage forms.

15. Propionyl L-carnitine, or a pharmaceutical salt thereof, for use in the prevention and/or treatment of left ventricular hypertrophy in ESRD or dialysed patients.

## Patentansprüche

1. Verwendung von Propionyl-L-carnitin oder eines pharmazeutisch akzeptablen Salzes davon zur Herstellung eines Medikaments zur Vorbeugung und/oder Behandlung von links ventrikulärer Hypertonie in ESRD oder Dialyse-Patienten.

2. Verwendung gemäß Anspruch 1, bei der das pharmazeutische Salz von Propionyl-L-carnitin ausgewählt ist aus folgender Gruppe: Chlorid, Bromid, Orotat, Aspartat, saures Aspartat, saures Zitrat, Magnesiumcitrat, Phosphat, saures Phosphat, Fumarat und saures Fumarat, Magnesiumfumarat, Lactat, Maleat und saures Maleat, Oxalat, saures Oxalat, Pamoat und saures Pamoat, Sulfat und saures Sulfat, Glucosephosphoat, Tartrat und saures Tartrat, Glicerophosphat, Mucat, Magnesiumtartrat, 2-Aminoethansulfonat, Magnesium-2-aminoetansulfonat, Methansulfonat, Colintartrat, Trichloroacetat und Trifluoroacetat.

3. Verwendung gemäß Anspruch 1, bei der Propionyl-L-carnitin dann verabreicht wird, wenn Hypertonie im wesentlichen nicht vorliegt oder nicht ernsthaft genug ist für eine medikamentöse Therapie.

4. Verwendung gemäß Anspruch 1, bei der Propionyl-L-carnitin dann verabreicht wird, wenn Hypertonie im wesentlichen vorliegt.

5. Verwendung gemäß Anspruch 1, bei der Propionyl-L-carnitin in Kombination mit Antihypertoniemitteln, die nicht selbst LVH behandeln, verwendet wird.

6. Verwendung gemäß Anspruch 5, bei der Antihypertoniemittel aus folgender Gruppe ausgewählt ist: Angiotensin-konvertierender Enzyminibitor, Kalziumkanalblocker, ACE-Inhibitoren, Diuretika, betaadrenerge Blocker oder Kalziumantagonisten.

7. Verwendung gemäß Anspruch 6, bei der ACE-Inhibitor ausgewählt ist aus folgender Gruppe: Spirapril, Enalapril, Ramipril, Perindopril, Indolapril, Lysinopril, Qhinapril, Pentopril, Cilazapril, Captopril, Zofenopril, Pivalopril und Fusinopril.

8. Verwendung gemäß Anspruch 6, bei dem die Kalziumantagonisten ausgewählt sind aus folgender Gruppe: Diltiazem, Nifedipin, Verapamil, Nicardipin und Nimodipin.

9. Verwendung gemäß Anspruch 1, bei der Propionyl-L-carnitin in einer Form vorliegt, die zur enteralen oder parenteralen Verabreichung geeignet ist.

10. Verwendung gemäß Anspruch 1, bei der Propionyl-L-carnitin in einer einmaligen oder geteilten Dosis von 1 bis ungefähr 5 g verabreicht wird, bevorzugt sind 2 bis 4 g/Tag bei oraler Verabreichung oder von ungefähr 1 bis 24 mg/kg/Körpergewicht, vorzugsweise 3 bis 6 mg/kg/Körpergewicht, am Ende jeder Dialysebehandlung bei intravenöser Verabreichung.

11. Verwendung gemäß Anspruch 9, bei der Propionyl-L-carnitin zur enteralen oder parenteralen Verabreichung in Form von Tabletten, Kapseln, Sirupen, Elixieren, Suppositorien, Suspensionen oder Lösungen vorliegt.

12. Verwendung gemäß Anspruch 5, bei der die Kombination durch eine einzige pharmazeutische Zusammensetzung verabreicht werden kann, die die aktiven Bestandteile in einem pharmazeutisch akzeptablen Hilfsstoff oder Träger kombiniert.

13. Verwendung gemäß Anspruch 5, bei der die zwei aktiven Bestandteile getrennt voneinander einer parallelen oder sequentiellen Art verabreicht werden können.

14. Verwendung gemäß Anspruch 13, bei der die zwei aktiven Bestandteile in jeder geeigneten Kombination von Dosierungsformen verabreicht werden können.

15. Propionyl-L-carnitin oder ein pharmazeutisch akzeptables Salz davon zur Verwendung in der Vorbeugung und/oder Behandlung von links ventrikulärer Hypertonie in ESRD oder Dialyse-Patienten.

## Revendications

1. Utilisation de propionyl L-carnitine, ou d'un sel pharmaceutique de celle-ci, pour la préparation d'un médicament destiné à la prévention et/ou au traitement d'une hypertrophie ventriculaire gauche dans l'IRSU ou chez des patients dialysés.

2. Utilisation selon la revendication 1, dans laquelle le sel pharmaceutique de proprionyl L-carnitine est choisi dans le groupe constitué par un chlorure, un bromure, un orotate, un aspartate, un aspartate acide, un citrate acide, un citrate de magnésium, un phosphate, un phosphate acide, un fumarate, et un fumarate acide, un fumarate de magnésium, un lactate, un maléate et un maléate acide, un oxalate, un oxalate acide, un pamoate et un pamoate acide, un sulfate et un sulfate acide, un glucose phosphate, un tartrate et un tartrate acide, un glycérophosphate, un mucate, un tartrate de magnésium, un 2-amino éthanesulfonate, un 2-amino éthanesulfonate de magnésium, un méthanesulfonate, un tartrate de choline, un trichloroacétate, et un trifluoroacétate.

3. Utilisation selon la revendication 1, dans laquelle la propionyl L-carnitine est administrée lorsqu'une hypertension essentielle n'est pas présente ou n'est pas suffisamment grave pour nécessiter une thérapie médicamenteuse.

4. Utilisation selon la revendication 1, dans laquelle la propionyl L-carnitine est administrée lorsqu'une hypertension essentielle est présente.

5. Utilisation selon la revendication 1, dans laquelle la propionyl L-carnitine est utilisée en combinaison avec des agents antihypertenseurs qui ne traitent pas eux-mêmes l'HVG.

6. Utilisation selon la revendication 5, dans laquelle l'agent antihypertenseur est choisi dans le groupe constitué par un inhibiteur de l'enzyme de conversion de l'angiotensine, un inhibiteur calcique, les inhibiteurs de l'ECA, les diurétiques, les bêtabloquants ou les antagonistes du calcium.

7. Utilisation selon la revendication 6, dans laquelle l'inhibiteur de l'ECA est choisi dans le groupe constitué par le spirapril, l'énalapril, le ramipril, le périndopril, l'indolapril, le lysinopril, le quinapril, le pentopril, le cilazapril, le captopril, le zofénopril, le pivalopril et le fosinopril.

8. Utilisation selon la revendication 6, dans laquelle l'antagoniste du calcium est choisi dans le groupe constitué par le diltiazem, la nifédipine, le vérapamil, la nicardipine et la nimodipine.

9. Utilisation selon la revendication 1, dans laquelle la propionyl L-carnitine est sous une forme convenable pour une administration entérale ou parentérale.

10. Utilisation selon la revendication 1, dans laquelle la propionyl L-carnitine est administrée en une dose unique ou en doses fractionnées de 1 à environ 5 g, sont préférées 2 à 4 g/jour lorsque administrée par voie orale, ou d'environ 1 à 24 mg/kg/poids corporel, préférées 3 à 6 mg/kg/poids corporel, à la fin de chaque session de dialyse, lorsque administrée par voie intraveineuse.

11. Utilisation selon la revendication 9, dans laquelle la propionyl L-carnitine pour une administration entérale ou parentérale est sous la forme de comprimés, capsules, sirops, élixirs, suppositoires, suspensions ou solutions.

12. Utilisation selon la revendication 5, dans laquelle la combinaison peut être administrée à partir d'une composition pharmaceutique unique qui combine les principes actifs dans un excipient et/ou un support pharmaceutiquement acceptable.

13. Utilisation selon la revendication 5, dans laquelle les deux principes actifs peuvent être administrés séparément dans une voie parallèle ou séquentielle.

14. Utilisation selon la revendication 13, dans laquelle les deux principes actifs peuvent être administrés dans n'importe quelle combinaison de formes posologiques convenable.

15. Propionyl L-carnitine, ou un sel pharmaceutique de celle-ci, pour une utilisation dans la prévention et/ou le traitement d'une hypertrophie ventriculaire gauche dans l'IRSU ou chez des patients dialysés.
